(19) Europäisches Patentamt
European Patent Office
Office européen des brevets

(11) Publication number: **0 140 154**
**B1**

(12) **EUROPEAN PATENT SPECIFICATION**

(45) Date of publication of patent specification: **22.02.89**

(51) Int. Cl.[4]: **C 07 D 249/08,**
A 01 N 43/653, A 61 K 31/41

(21) Application number: **84111417.6**

(22) Date of filing: **25.09.84**

# (54) 1,2,4-Triazolylpropanols, and their production and use.

(30) Priority: **26.09.83 JP 178914/83**
**27.08.84 JP 177956/84**

(43) Date of publication of application:
**08.05.85 Bulletin 85/19**

(45) Publication of the grant of the patent:
**22.02.89 Bulletin 89/08**

(84) Designated Contracting States:
**AT BE CH DE FR GB IT LI NL SE**

(58) References cited:
**EP-A-0 061 835**
**EP-A-0 070 798**
**EP-A-0 095 828**
**EP-A-0 098 942**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Saji, Ikutaro**
**Q-401, Aobaokaminami 8-chome**
**Suita-shi Osaka-fu (JP)**
Inventor: **Ohuchi, Renzo**
**10-3-326, Sonehigashimachi**
**Toyonaka-shi Osaka-fu (JP)**
Inventor: **Ichise, Katsuaki**
**2-30-310, Nagaoka 2-chome**
**Nagaokakyo-shi Kyoto-fu (JP)**
Inventor: **Tanio, Tomoharu**
**E1-106, Takenodai 2-ban**
**Nagaokakyo-shi Kyoto-fu (JP)**
Inventor: **Okuda, Takao**
**10-3-337, Sonehigashimachi 2-chome**
**Toyonaka-shi Osaka-fu (JP)**
Inventor: **Atsumi, Toshio**
**3-45, Seiwadainishi 2-chome**
**Kawanishi-shi Hyogo-ken (JP)**

(74) Representative: **Vossius & Partner**
**Siebertstrasse 4 P.O. Box 86 07 67**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 140 154 B1**

## Description

The present invention relates to 1,2,4-triazolylpropanols, and their acid addition salts.

The 1,2,4-triazolylpropanols of this invention are representable by the formula:

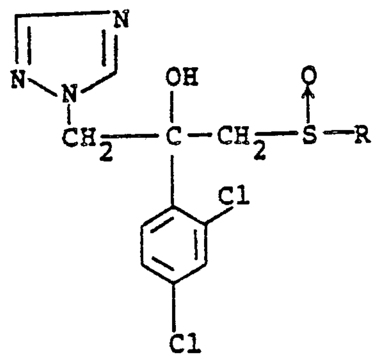

(I)

wherein R is a straight or branched $C_5$ alkyl group (e.g. n-pentyl, isopentyl, sec-pentyl, tert-pentyl, 1-methylbutyl, 2-methylbutyl 2,2-dimethylpropyl). Their acid addition salts are also included in the scope of the invention.

The 1,2,4-triazolylpropanols (I) of the invention contain at least two asymmetric centers so that there exist two or more diastereomers, which can be separated by a per se conventional separation procedure such as chromatography. Each diastereomer may further be resolved into optical isomers by a per se conventional procedure. These stereoisomers, irrespective of being isolated or not, are also included in the scope of the invention.

The 1,2,4-triazolylpropanols (I) as well as their acid addition salts and stereoisomers are novel and useful as pharmaceuticals, particularly for controlling fungal infections such as mycosis in mammals including human beings.

In recent years, fungal infections such as mycosis increase to cause a great problem in the medical field. Especially deep mycosis show rapid increase with the development of antibacterial agents, and opportunistic infections caused by mycocytes originally not having any substantial pathogenecity are observed frequently. Also, the administration of anticancer agents or immunosuppressive agents to cancerous, leukemic or immunological diseases often induce serious mycosis, which may rather be the direct cause to death.

For treatment or control of deep mycosis, there is widely used amphotericin B. However, this drug has a problem in toxicity, and certain restriction is present on its use. There is also used griseofulvin as an oral drug for superficial mycosis such as tinea. However, carcinogenecity was observed on this drug, and since then, its use had been considerably restricted. In view of the above circumstances, the appearance of a novel drug for treatment of fungal diseases, preferably orally applicable, has been much demanded.

Under the situation as above, many studies have been made on synthetic anti-fungal agents of azole type. As the result, ketoconazole has been developed as an antifungal agent and is widely used in western countries. This drug can be administered by oral route and shows a broad spectrum against various fungi. Advantageously, its toxicity is much lower than that of amphotericin B. However, it is still not a satisfactory anti-fungal agent. When, for instance, it is to be administered to human beings, care must be taken on its toxicity to liver. Further, its anti-fungal efficacy is not sufficiently high. Accordingly, the appearance of an anti-fungal agent having a stronger anti-fungal activity and a lower toxicity has still been desired.

As the result of an extensive study aiming at development of any new anti-fungal agent applicable through oral route, it has been found that the 1,2,4-triazolylpropanols (I) show a strong anti-fungal activity against a wide variety of fungi including Candida, Aspergillus, Trichophyton, Cryptococcus, etc. It has also been found that their continuous use increases markedly their antifungal effect. It has further been found that they exhibit high anti-fungal potency with low acute toxicity and less influence onto liver. This invention is based on the above findings and can provide an anti-fungal agent having a high chemotherapeutic index.

Japanese Patent Publn. (unexamined) No. 185571/83 (corresponding to EP—A—95828) discloses 1,2,4-trialzols covering generically the 1,2,4-triazolylpropanols (I). Further, it is stated in this literature that those 1,2,4-triazoles are effective in treatment of fungal infections in mammals including human beings. Among various compounds, 3-methylsulfonyl-2-(2,4-dichlorophenyl)-1-(1H-1,2,4-triazol-1-yl)propan-2-ol is stated to be particularly favorable. However, said literature does not specifically disclose the 1,2,4-triazolylpropanols (I). Naturally, it is entirely silent on the anti-fungal activity of the 1,2,4-triazolylpropanols (I). Quite advantageously, the 1,2,4-triazolylpropanols (I) of the invention show a stronger antifungal activity and a much lesser toxicity than 3-methylsulfonyl-2-(2,4-dichlorophenyl)-1-(1H-1,2,4-triazol-1-yl)propan-2-ol of said literature does. Particularly notable is that said 3-methylsulfonyl-2-(2,4-dichlorophenyl)-1-(1H-1,2,4-triazol-1-yl)propan-2-ol is a sulfonyl compound, while the 1,2,4-triazolylpropanols (I) of the invention are

sulfinyl compounds, and the sulfinyl compounds according to the invention are much stronger than the corresponding sulfonyl compounds according to said literature in anti-fungal potency.

The 1,2,4-triazolylpropanols (I) may be produced by either one of the following procedures:

Procedure (a)

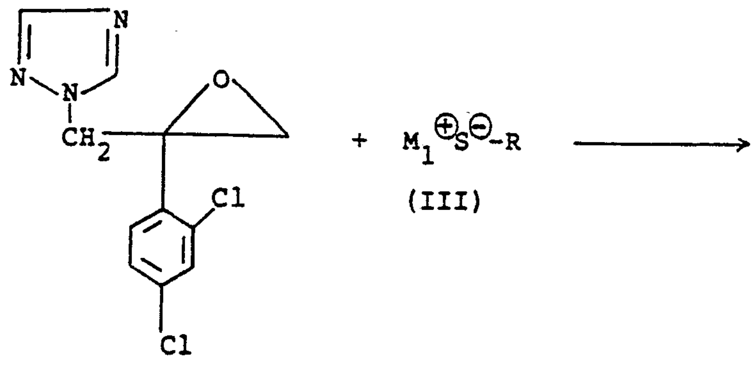

(III)

(II)

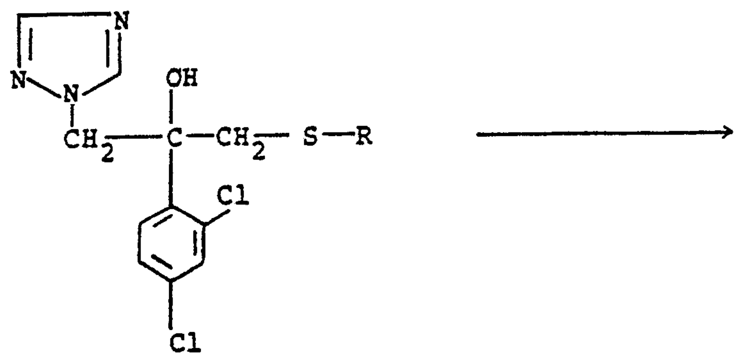

(I)

(IV)

wherein $M_1$ is an alkali metal atom (e.g. sodium, potassium, lithium) and R is as defined above.

Namely, the epoxy compound (II), which is known, is first reacted with an alkali metal salt of the thiol compound (III) to give the sulfide compound (IV), which is then oxidized to give the 1,2,4-triazolylpropanol (I).

The reaction between the epoxy compound (II) and the alkali metal salt of the thiol compound (III) may be carried out in an inert solvent such as an ether (e.g. tetrahydrofuran) or dimethylformamide at a temperature of 0°C to the boiling temperature of the solvent, preferably of 10 to 60°C. Usually, the alkali metal salt of the thiol compound (III) is used in an amount of 1 to 2 equivalents to the epoxy compound (II).

The oxidation of the sulfide compound (IV) may be carried out by treatment with an oxidizing agent such as a peracid (e.g. m-chloroperbenzoic acid) in an inert solvent such as a halogenated hydrocarbon (e.g. chloroform) at a temperature of −30°C to the boiling temperature of the solvent, preferably of 0°C to room temperature. The amount of the oxidizing agent is usually about an equivalent to the sulfide compound (IV).

Procedure (b)

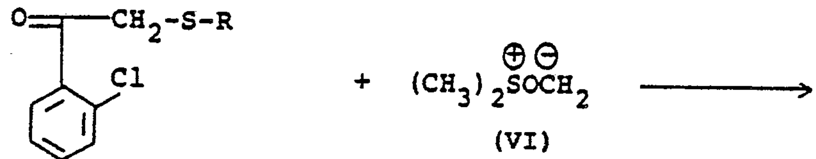

(VI)

(V)

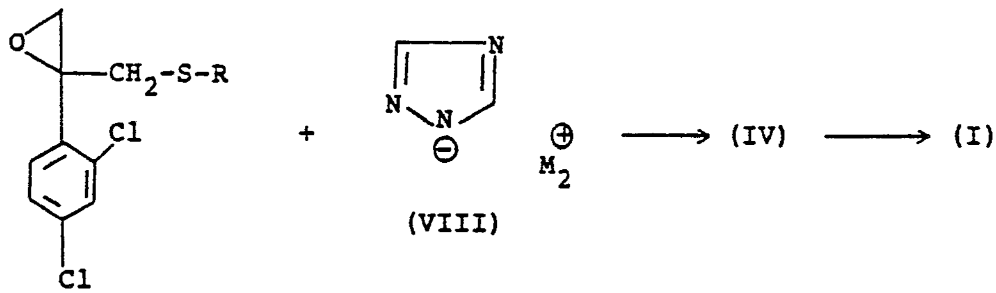

(VIII) (IV) (I)

(VII)

wherein $M_2$ is an alkali metal atom (e.g. sodium, potassium) and R is as defined above.

Namely, the ketone compound (V) (U.S. patent 4,128,581) is first reacted with the dimethyloxosulfonium methylide (VI), and the resultant sulfide compound (VII) is then reacted with an alkali metal salt of the 1,2,4-triazole (VIII) to give the sulfide compound (IV), which is oxidized to the 1,2,4-triazolylpropanol (I).

The reaction between the ketone compound (V) and the dimethyloxosulfonium methylide (VI) may be effected in an inert solvent (e.g. dimethylsulfoxide) at a temperature of 30 to 60°C. The amount of the dimethyloxosulfonium methylide (VI) is usually from 1 to 5 equivalents, preferably from 1 to 2 equivalents, to the ketone compound (V).

The subsequent reaction of the sulfide compound (VII) with the alkali metal salt of the 1,2,4-triazole (VIII) is usually carried out in an inert solvent (e.g. dimethylformamide) at a temperature of from room temperature to the boiling point of the solvent, preferably of 70 to 120°C. The amount of the alkali metal salt of the 1,2,4-triazole (VIII) is usually from 1 to 5 equivalents, preferably from 1 to 3 equivalents, to the sulfide compound (VII).

The acid addition salts of the 1,2,4-triazolylpropanols (I) may be prepared by treatment of the 1,2,4-triazolylpropanol (I) with an acid in a per se conventional procedure for salt formation. Examples of the acid are inorganic acids (e.g. hydrochloric acid, hydrobromic acid, phosphoric acid, nitric acid, sulfuric acid) and organic acids (e.g. oxalic acid, acetic acid, maleic acid, succinic acid, fumaric acid, tartaric acid, citric acid, salicylic acid, sorbic acid, lactic acid). The acid addition salts are preferred to be physiologically acceptable.

The 1,2,4-triazolylpropanols (I) and their physiologically acceptable acid addition salts can be administered as such or together with any excipient or additive such as a carrier, a diluent or a dispersant orally or parenterally in the form of conventional pharmaceutical preparations. For instance, they can be administered in the form of conventional solid or liquid pharmaceutical preparations such as solutions, suspensions, powders, granules, capsules, tablets, injections, ointments, tinctures, etc. The daily dosage may vary depending upon the state of infection, the age and body weight of patients, the administration route, etc., and the normal oral dosage to a human adult is between 50 and 200 mg, preferably between 100 and 600 mg, dividing in one to several times per day.

Practical and presently preferred embodiments of the invention are illustratively shown in the following examples.

Reference Example 1

A 60% oily dispersion of sodium hydride (0.89 g; 22.2 mmol) was added to dimethylformamide (20 ml), and 1-pentanethiol (2.75 ml; 22.2 mmol) was dropwise added thereto. To the resultant mixture, a solution of 2-(2,4-dichlorophenyl)-2-(1,2,4-triazol-1-yl)methyloxirane (3.0 g; 11.1 mmol) in dimethylformamide (5 ml) was dropwise added. The resultant mixture was stirred at room temperature for 3 hours, and the solvent was removed by distillation. Water was added thereto, and the resulting mixture was extracted with chloroform. The chloroform extract was washed with water, dried over magnesium sulfate and concentrated under reduced pressure. The oily residue was subjected to silica gel chromatography using chloroform as an eluent to give 2-(2,4-dichlorophenyl)-3-n-pentylthio-1-(1,2,4-triazol-1-yl)propan-2-ol (3.39

g). Yield, 81.5%.

NMR ($CDCl_3$) δ: 0.86 (3H, t), 1.0—1.6 (6H, m), 2.25 (2H, t), 2.90 (1H, d, J=14 Hz), 3.64 (1H, d, J=14 Hz), 4.60 (1H, s), 4.70 (1H, d, J=14 Hz), 5.0 (1H, d, J=14 Hz), 7.12—7.70 (3H, m), 7.80 (1H, s), 8.00 (1H, s).

The oxalate gives a melting point of 83 to 86°C.

Example 1

To a solution of 2-(2,4-dichlorophenyl)-3-n-pentylthio-1-(1,2,4-triazol-1-yl)propan-2-ol (1.46 g; 3.9 mmol) in chloroform (30 ml), m-chloroperbenzoic acid (0.67 g; 3.9 mmol) was gradually added, and the resulting mixture was stirred at room temperature for 3 hours. The reaction mixture was poured into ice-water, made basic with ammonia water and extracted with chloroform. The extract was washed with water, dried over magnesium sulfate and concentrated. The oily residue was subjected to silica gel column chromatography. The column was eluted with chloroform. The eluate was treated with an etheral solution of oxalic acid to give 2-(2,4-dichlorophenyl)-3-n-pentylsulfinyl-1-(1,2,4-triazol-1-yl)propan-2-ol oxalate (Compound No. 1A) (0.84 g). Yield, 44.8%. M.P., 65 to 68°C. Further elution with chloroform gave the diastereomer (Compound No. 1B) (0.3 g). Yield, 19.7% M.P., 117 to 119°C.

Reference Example 2

A 60% oily dispersion of sodium hydride (2.97 g; 74.2 mmol) was added to dimethylformamide (60 ml), and tert-pentylmercaptan (7.73 g; 74.2 mmol) was dropwise added thereto while cooling with ice. The resultant mixture was stirred at room temperature for 1 hour. A solution of 2-(2,4-dichlorophenyl)-2-(1,2,4-triazol-1-yl)methyloxirane (10.02 g; 37.1 mmol) in dimethylformamide (15 ml) was dropwise added thereto under ice-cooling. The resultant mixture was stirred at room temperature for 3 hours and poured into ice-water. After addition of sodium chloride thereto, the resulting mixture was extracted with ether. The ether extract was washed with an aqueous sodium chloride solution, dried over magnesium sulfate and concentrated under reduced pressure. The oily residue was subjected to medium pressure chromatography using chloroform as an eluent to give 2-(2,4-dichlorophenyl)-3-tert-pentylthio-1-(1,2,4-triazol-1-yl)propan-2-ol (11.40 g) as an oil. Yield, 82.1%.

NMR ($CDCl_3$) δ: 0.80—1.69 (11H, m), 2.88 (1H, d, J=13 Hz), 3.62 (1H, d, J=13 Hz), 4.38 (1H, s), 4.81 (1H, d, J=14 Hz), 4.97 (1H, d, J=14 Hz), 7.12—7.68 (3H, m), 7.82 (1H, s), 8.07 (1H, s).

Example 2

To a solution of 2-(2,4-dichlorophenyl)-3-tertpentylthio-1-(1,2,4-triazol-1-yl)propan-2-ol (11.40 g; 30.5 mmol) in chloroform (84 ml), m-chloroperbenzoic acid (5.26 g; 30.5 mmol) was gradually added under ice-cooling, and the resulting mixture was stirred at room temperature for 1.5 hours. The reaction mixture was poured into ice-water, made basic with ammonia water and extracted with chloroform. The chloroform was washed with water, dried over magnesium sulfate and concentrated under reduced pressure. The oily residue was subjected to medium pressure chromatography using a mixture of chloroform and methanol (50:1) as an eluent. The oily product was crystallized from n-hexane and recrystallized from a mixture of n-hexane and ethyl acetate to give 2-(2,4-dichlorophenyl)-3-tertpentylsulfinyl-1-(1,2,4-triazol-1-yl)propan-2-ol (Compound No. 2A) (5.86 g). Yield, 49.3% M.P. 112.5—114.5°C.

NMR ($CDCl_3$) δ: 0.80—1.68 (11H, m), 2.58 (1H, d, J=13 Hz), 3.88 (1H, d, J=13 Hz), 4.42 (1H, d, J=14 Hz), 4.94 (1H, d,d, J=14 Hz, J'=2 Hz), 5.91 (1H, d, J=2 Hz), 7.28—7.46 (2H, m), 7.89 (1H, d, J=9 Hz), 8.25 (1H, s).

In the same manner as in Example 1 or 2, the 1,2,4-triazolylpropanols (I) as shown in Table 1 were produced:

Table 1

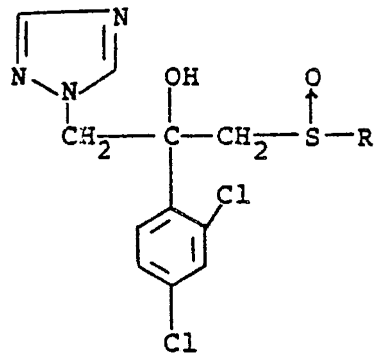

(I)

| Example No. | Compound No. | R | Melting point (°C) |
|---|---|---|---|
| 3 | 3A<br>3B | 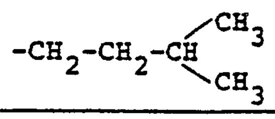 | 87 - 89<br>125 - 127 |
| 4 | 4A<br>4B | 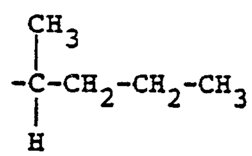 | 107 - 110<br>142 - 144 |
| 5 | 5A<br>5B | 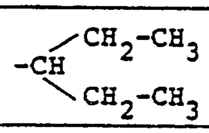 | 107 - 108<br>133 - 135 |

As stated above the 1,2,4-triazolylpropanols (I) exhibit a strong antifungal activity against a wide variety of fungi with low toxicity to mammals. In order to support such characteristic property, some typical test examples are set forth below.

Test Example 1

Antifungal activity test (in vivo):—

Candida albicans KB—8 as pre-cultured at 30°C for 4 days was suspended in physiologically saline solution, and the suspension was intravenously injected into DDY strain male mice of 5 weeks old, every 10 animals being grouped, through the tail vein so as to inoculate a cell number of $10^7$ per animal.

The animals were orally medicated with the test compound in the form of 0.5% methylcellulose suspension at a total dose of 20 mg per kg of the body weight 0, 5, 24 and 48 hours after the infection, and observation was continued for 10 days after the injection. The results are shown in Table 2, wherein the numeral indicates an average number of survival days of 10 animals as one group in contrast to that of the non-medicated (control) group.

For comparison, the following compounds were also tested:

| No. | Chemical structure | Remarks |
|---|---|---|
| I | 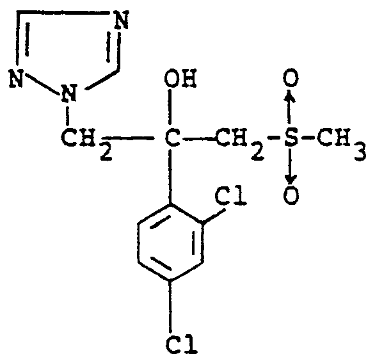 | Japanese Patent Publn. (unexamined) No. 185571/83; Example 19 |
| II | 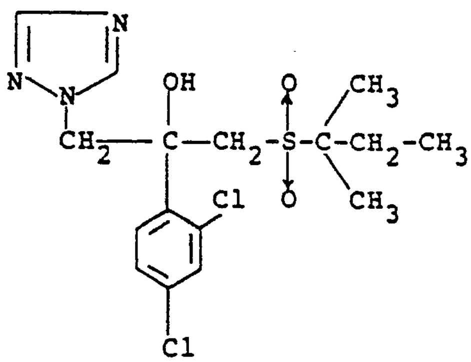 | |
| Ketoconazole | 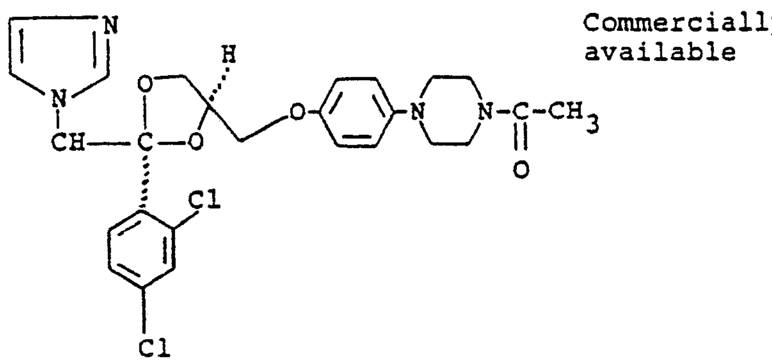 | Commercially available |

Table 2

| Compound No. | Average survival days (control) |
|---|---|
| 1A | 9.0 (1.2) |
| 2A | 9.7 (1.2) |
| 3A | 7.7 (1.2) |
| 4A | 9.2 (1.2) |
| 5A | 8.9 (1.2) |
| I | 9.6 (1.2) |
| II | 1.4 (1.0) |
| Ketoconazole | 4.8 (0.8) |

Test Example 2

Antifungal activity test (in vivo);—

Aspergillus fumigatus MTU 16001 or Cryptococcus neoformans MTU 13001 as pre-cultured at 30°C for 4 days was suspended in physiologically saline solution, and the suspension was intravenously injected into DDY strain male mice of 5 weeks old (body weight, 24—27 g), every 10 animals being grouped, through the tail vein so as to inoculate a cell number of 2 × $10^6$ per animal.

The animals were orally medicated with the test compound in the form of 0.5% methylcellulose suspension at a total dose of 20 mg per kg of the body weight 0, 5, 24 and 48 hours after the infection, and observation was continued for 10 days after the injection. The results are shown in Table 3, wherein the numeral indicates an average number of survival days of 10 animals as one group in contrast to that of the non-medicated (control) group.

Table 3

| Compound No. | Aspergillus fumigatus | | Cryptococcus neoformans | |
|---|---|---|---|---|
| | Survival rate at complete death of non-medicated group (%) | Average survival day(s) | Survival rate at complete death of non-medicated group (%) | Average survival day(s) |
| 2A | 70 | 3.6 | 90 | 8.6 |
| I | 60 | 2.7 | 80 | 7.8 |
| Control | 0 | 1.0 | 0 | 1.6 |

Test Example 3

Acute toxicity:—

DDY strain male mice of 5 weeks old, every 10 mice being grouped, received orally the test compound in the form of 0.5% methylcellulose suspension at a dose from five kinds increasing with a common ratio of 1.3, and observation on the death of animals was continued. Seven days after the medication, the $LD_{50}$ value was calculated from the rate of death according to the Probit method. The results are shown in Table 4.

Table 4

| Compound No. | $LD_{50}$ (mg/kg) |
|---|---|
| 1A | 1743 |
| 2A | 1827 |
| 3A | >2000 |
| 4A | >1500 |
| 5A | >2000 |
| I | 770 |
| Ketoconazole | 1102 |

Test Example 4

Liver weight increase test:—

Candida albicans KB—8 was intravenously injected into DDY strain male mice of 5 weeks old, every 20 animals being grouped, at a rate of 2 × $10^5$ cells per mouse. After injection, the animals were orally medicated with the test compound in the form of 0.5% methylcellulose suspension once a day for 20 consecutive days, during which observation on the liver weight was continued.

The average liver weight in survived animals as calculated are shown in Table 5, from which it is understood that the liver weight is not increased when the compound of the invention is given at a daily dose of not more than 4 mg/kg while the liver weight is increased when the compound of Japanese Patent Publn. (unexamined) No. 185571/83 is given at a daily dose of not less than 1 mg/kg.

Table 5

| Compound No. | Dose (mg/kg, p.o.) | Average weight of liver (g) |
|---|---|---|
| 1A | 0.5<br>1.0<br>2.0<br>4.0<br>8.0 | 1.20<br>1.18<br>1.15<br>1.25<br>1.60 |
| I | 0.5<br>1.0<br>2.0<br>4.0<br>8.0 | 1.30<br>1.50<br>1.70<br>1.85<br>1.90 |
| Control | 0 | 1.20 |

**Claims**

1. A 1,2,4-triazolylpropanol of the formula:

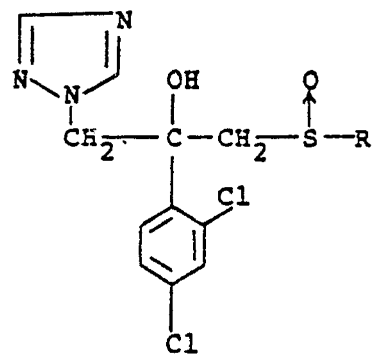

(I)

wherein R is a straight or branched $C_5$ alkyl group, and an acid addition salt thereof.

2. The 1,2,4-triazolylpropanol and an acid addition salt thereof according to claim 1, wherein R is a tert-pentyl group.

3. A process for producing the 1,2,4-triazolylpropanol and an acid addition salt thereof according to claim 1, which comprises reacting (a) the epoxy compound of the formula:

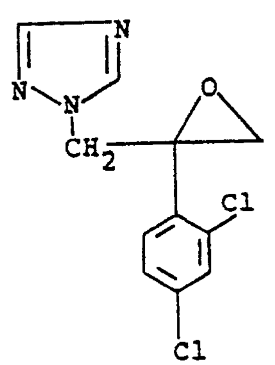

with an alkali metal salt of the thiol compound of the formula:

$$M_1^{\oplus}S^{\ominus}R$$

wherein $M_1$ is an alkali metal atom and R is as defined in claim 1, and oxidizing the resulting sulfide

compound of the formula:

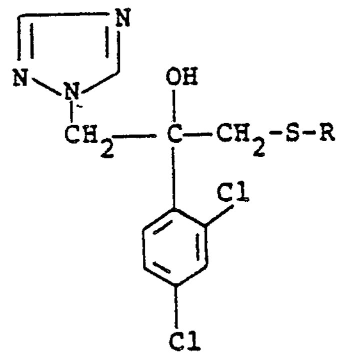

wherein R is as defined in claim 1 or (b) reacting the epoxy compound of the formula:

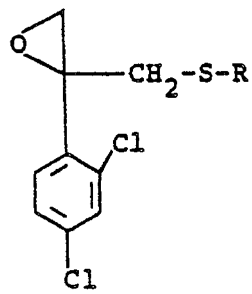

wherein R is as defined in claim 1 with an alkali metal salt of the 1,2,4-triazole of the formula:

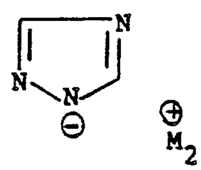

wherein $M_2$ is an alkali metal atom, and oxidizing the resultant sulfide compound of the formula:

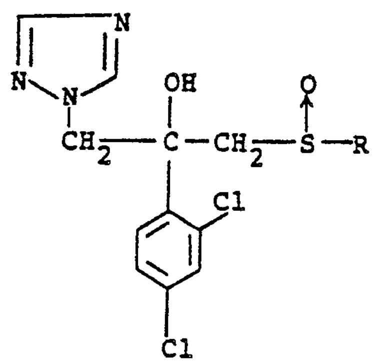

wherein R is as defined in claim 1.

4. An antifungal composition which comprises an antifungally effective amount of at least one of the 1,2,4-triazolylpropanols and their acid addition salts according to claim 1 as an active ingredient and a pharmaceutically acceptable carrier or diluent.

5. A 1,2,4-triazolylpropanol or its pharmaceutically acceptable salts according to claim 1 for use as an antifungal agent.

**Patentansprüche**

1. Ein 1,2,4-Triazolylpropanol der Formel

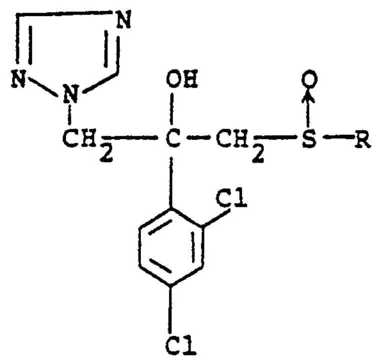

(I)

in der R ein verzweigter oder unverzweigter $C_5$-Alkylrest ist und dessen Säureadditionssalze.

2. 1,2,4-Triazolylpropanol und dessen Säureadditonssalze nach Anspruch 1, in denen R eine tert.-Pentylgruppe ist.

3. Verfahren zur Herstellung des 1,2,4-Triazolylpropanols und seiner Säureadditionssalze nach Anspruch 1, bei dem man

(a) die Epoxyverbindung der Formel

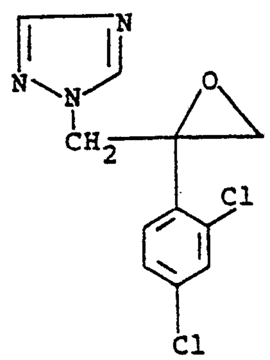

mit einem Alkalimetallsalz der Thiolverbindung der Formel

$$M_1^{\oplus}S^{\ominus}R$$

in der $M_1$ ein Alkalimetallatom ist und R die in Anspruch 1 angegebene Bedeutung hat, umsetzt und die so erhaltene Sulfidverbindung der Formel

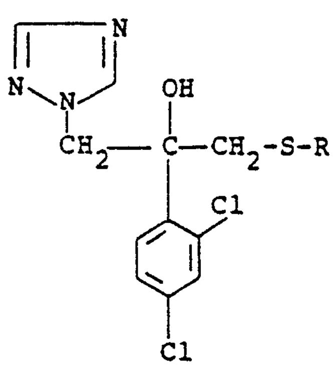

in der R die in Anspruch 1 angegebene Bedeutung hat, oxidiert, oder bei dem man

(b) die Epoxyverbindung der Formel

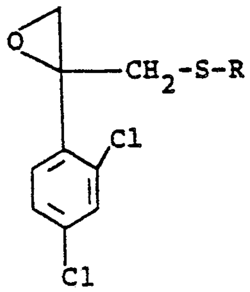

in der R die in Anspruch 1 angegebene Bedeutung hat, mit einem Alkalimetallsalz des 1,2,4-Triazols der Formel

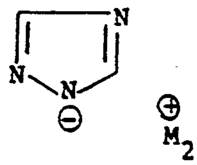

in der $M_2$ ein Alkalimetallatom ist, umsetzt und die so erhaltene Sulfidverbindung der formel

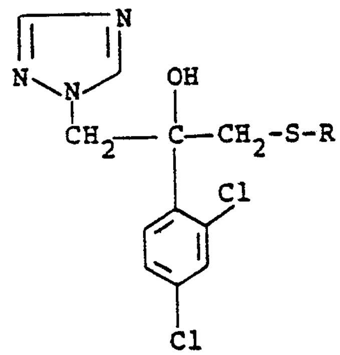

in der R die in Anspruch 1 angegebene Bedeutung hat, oxidiert.

4. Fungizides Mittel, enthaltend eine fungizid wirksame Menge von mindestens einem der 1,2,4-Triazolylpropanole oder ihrer Säureadditionssalze nach Anspruch 1 als Wirkstoff und ein pharmazeutisch verträgliches Trägermaterial oder Verdünnungsmittel.

5. 1,2,4-Triazolylpropanol oder seine pharmazeutisch verträglichen Salze nach Anspruch 1 zur Verwendung als Fungizid.

**Revendications**

1. Un 1,2,4-triazolylpropanol de formule:

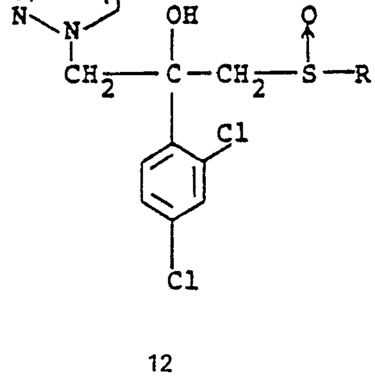

(I)

dans laquelle R est un groupe alkyle en $C_5$ à chaîne droite ou ramifiée, et un de ses sels d'addition d'acides.

2. Le 1,2,4-triazolylpropanol et un de ses sels d'addition d'acides selon la revendication 1, caractérisés en ce que R est un groupe tert-pentyle.

3. Un procédé pour fabriquer le 1,2,4-triazolylpropanol et ses sels d'addition d'acides selon la revendication 1, caractérisé en ce qu'il consiste à faire réagir (a) le composé époxy de formule:

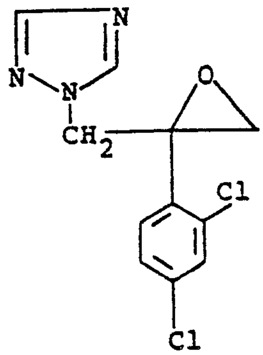

avec un sel de métal alcalin du composé thiol de formule:

$$M_1^{\oplus}S^{\ominus}R$$

dans laquelle $M_1$ est un atome de métal alcalin et R est tel que défini à la revendication 1, et à oxyder le composé sulfure résultant de formule:

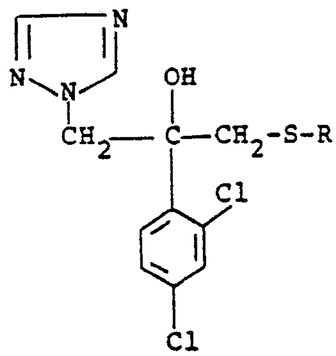

dans laquelle R est tel que défini à la revendication 1, ou (b) à fait réagir le composé époxy de formule:

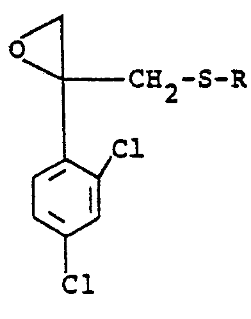

dans laquelle R est tel que défini à la revendication 1 avec un sel de métal alcalin du 1,2,4-triazole de formule:

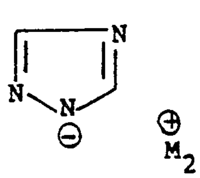

dans laquelle $M_2$ est un atome de métal alcalin et à oxyder le composé sulfur résultant de formule:

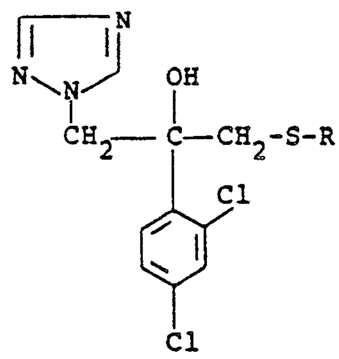

dans laquelle R est tel que défini à la revendication 1.

4. Une composition antifongique, caractérisée en ce qu'elle contient une quantité efficace comme antifongique d'au moins un des 1,2,4-triazolylpropanols et de leurs sels d'addition d'acides selon la revendication 1 comme ingrédient actif et un support ou diluant acceptable en pharmacie.

5. Un 1,2,4-triazolylpropanol ou ses sels acceptables en pharmacie selon la revendication 1 pour l'utilisation comme agent antifongique.